# EUROPEAN PATENT SPECIFICATION

(11) **EP 4 351 689 B1**
(45) Date of publication and mention of the grant of the patent: **07.05.2025**
(21) Application number: 22729721.5
(22) Date of filing: 23.05.2022
(51) Int. Cl.: A61M 25/00

(54) **CATHETER FOR CEREBRAL ANGIOGRAPHY AND NEUROINTERVENTIONS**
KATHETER FÜR ZEREBRALE ANGIOGRAFIE UND NEUROINTERVENTIONEN
CATHÉTER POUR ANGIOGRAPHIE CÉRÉBRALE ET NEURO-INTERVENTIONS

(30) Priority: 11.06.2021 EP 21382519
(43) Date of publication of application: 17.04.2024
(73) Proprietor: Barranco Pons, Roger, 08640 Olesa de Montserrat (ES)
(72) Inventor: Barranco Pons, Roger, 08640 Olesa de Montserrat (ES)
(74) Representative: Torner, Juncosa I Associats, SL
(86) International application number: PCT/EP2022/063840
(87) International publication number: WO 2022/258353

(56) References cited:
- WO-A1-2012/130878
- WO-A1-2020/072895
- US-A1- 2006 089 618
- US-A1- 2006 161 177
- US-A1- 2009 082 756

## Description

### Technical Field

The present invention is directed, in general, to the field of catheters. In particular, the invention relates to a catheter for cerebral angiography and neurointerventions.

### Background of the Invention

Transfemoral access (TFA) has been widely used in neurointerventional procedures; it is the vascular approach most frequently used for catheterization of the supraaortic and intracranial vessels. However, TFA can lead to potentially life-threatening complications, which has sparked interest in transradial access (TRA) as a safer access option.

In a review of 19,826 consecutive patients undergoing diagnostic cerebral angiography using TFA, access-site hematoma was the most common complication overall (4.2%).

Percutaneous radial access was described by Campeau in 1989 for coronary angiography in 100 patients. Since then, a growing body of evidence suggests that TRA is safer for patients and more cost-effective.

In the 2018 ESC/EACTS guidelines on myocardial revascularization, radial access is preferred for any PCI irrespective of clinical presentation, unless there are overriding procedural considerations.

During all this years, technical innovations and specific TRA catheters for gaining access to the coronary arteries have helped to change the TFA to TRA in the cardiology field. Likewise, TRA access is showing to be a good choice for elderly patients to perform stroke thrombectomy or other kinds of neuro vascular diseases.

Though TRA has become progressively accepted for diagnostic cerebral angiography and neurointerventional procedures, there is still a need for proper specific materials to perform neurointerventions through TRA.

The known standard catheters in the field, such as Cobra catheters or Simmons catheters when used in the left vertebral artery show difficulties in its manipulation, not being able to rise in the left vertebral artery but tending to fall into the descending aorta. This also may happen with the other supraaortic vessels depending on the anatomy of the aortic arch.

Besides, International patent application WO2020072895 discloses a cardiac catheter for angiographic procedures of a left internal mammary artery bypass graft performed using a right radial arterial approach. The catheter includes a guide catheter comprising an elongated member having an outer sheath body portion that includes (see Figs. 3A and 3C) an outer sheath proximal end, an outer sheath distal end, and an outer sheath lumen extending through the outer sheath between the outer sheath proximal end and the outer sheath distal end. The outer sheath includes a curved section that has: a first curved segment, a second curved segment, a third curved segment, a fourth curved segment, a first extended segment between the third curved segment and the fourth curved segment, and a second extended segment between the fourth curved segment and the outer sheath distal end.

US2009082756A1 provides a catheter suitable for catheterization of a right coronary artery using a right transradial approach. The catheter includes a tip near its distal end, a primary curve, a secondary curve, a tertiary curve and a proximal end accessible external a catheterized body. When properly inserted, the secondary curve may rest near the junction of the bracheocephalic trunk and the right subclavian artery, and the tertiary curve may rest in the superior curve of the right subclavian artery. In addition to improved directionality, the secondary and/or tertiary curve offers additional resistance against cephalic, or upward, back-up force by providing caudal, or downward, torque to more distal portions of the catheter body. This catheter is thus designed and used for internal mammary catheterization (the artery used to perform bypass to coronary(heart arteries)) and not to catheterize brain arteries (carotids and vertebral arteries) from a radial approach as the proposed catheter for cerebral angiography and neurointerventions.

US2006089618 discloses a catheter having a distal section incluidng a first straight section M of the shaft, a first curved section N of the shaft extending distally from the first straight section M, a second straight section O of the shaft extending distally from the first curved section N, a second curved section P of the shaft extending distally from the second straight section O, a third straight section Q of the shaft extending distally from the second curved section P, a third curved section R of the shaft extending distally from the third straight section Q, and a fourth straight section S of the shaft extending distally from the third curved section R.US2006161177 discloses a catheter comprising at least one straight proximal section; and at least two curved sections with opposite curvature. The straight and curved sections collectively have a length and shape to dispose the distal tip at or near the coronary sinus when accessed from the right subclavian vein.

More specific catheters to perform supraaortic vessels catheterization from a TRA access are therefore needed.

### Description of the Invention

To that end the present invention provides a catheter for cerebral angiography and neurointerventions.

The proposed catheter needs to perform retrogradely catheterization to supra-aortic vessels meanwhile having a contact/support to the lower aspect of the aortic arch, this will allow not only catheterize the desired vessel, it will allow the advancement of the catheter through a guidewire (once the vessel is catheterized) by having contact of the catheter in the lower aspect of the aortic arch (if the catheter does not have the contact, while trying to advance it, it will fall in the descending aorta).

The proposed catheter is defined in claim 1. It comprises: a proximal end, a distal end and a plurality of segments. The plurality of segments include a first curved segment, a second curved segment and a third linear segment.

The first curved segment has a curve with a first radius of curvature and is extended with a first linear segment. The second curved segment connects the first linear segment with a second linear segment and has a curve with a second radius of curvature. The curve of the first curved segment is in a different direction than the curve of the second curved segment, or in other words, the concave portion of the second curved segment faces an area delimited by the first linear segment and the second linear segment. The second linear segment has a length greater than a length of the first linear segment.

In the proposed catheter the third linear segment is connected to an end of the second linear segment through a curved portion that orients the third linear segment in a direction that approaches towards the first curved segment.

In some embodiments, the curved portion comprises a radius of curvature that is smaller than the first radius of curvature.

In some embodiments, the second linear segment and the first linear segment are approximately parallel one to another.

In some embodiments, the intersection of the third linear segment and the second linear segment covers an angle of at least 160º.

Particularly, the first radius of curvature is smaller than the second radius of curvature. The proposed catheter can have different lengths. Particularly, the total length of the catheter is between 80 cm and 150 cm. Moreover, in some embodiments, the second linear segment can have a length comprised in a range between 3 - 10 cm and the third linear segment can have a length comprised in a range between 70-147 cm.

The proposed catheter device enables going up in the left vertebral artery without dropping into the descending aorta while offering an improved handling and control. Likewise, the catheter provides a good torque and pushability combination.

The foregoing describes embodiments of the present invention. Modifications obvious to those skilled in the art can be made thereto, without departing from the scope of the present invention.

### Brief Description of the Drawings

The previous and other advantages and features will be more fully understood from the following detailed description of embodiments, with reference to the attached figures, which must be considered in an illustrative and non-limiting manner, in which:

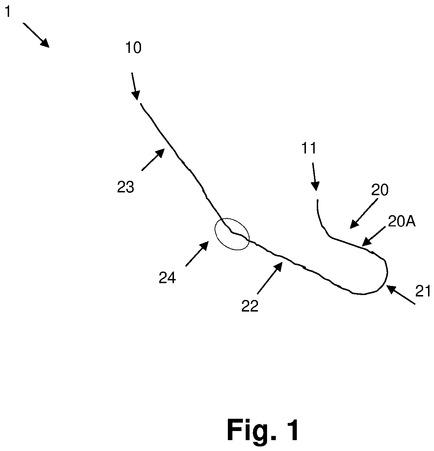

Fig. 1 schematically illustrates a catheter for cerebral angiography and neurointerventions, according to an embodiment of the present invention.

### Detailed Description of Preferred Embodiments

Fig.1 shows a schematic illustration of the proposed catheter 1. Particularly, the catheter 1 is used for cerebral angiography and neurointerventions.

According to the embodiment of Fig. 1, the catheter 1 has a proximal end 11, a distal end 10 and a series of segments 20, 21, 22, 23 connecting said proximal end 11 and distal end 10. Although not illustrated in the figure, in some embodiments, the proposed catheter 1 also comprises an outer sheath, for example a 2.7-2.8 outer diameter for 6F sheath, among others.

As shown in Fig.1 a first curved segment 20 comprises a curve with a first radius of curvature and extends with a first linear segment 20A. A second curved segment 21 extends from the first segment 20 and has a curve with a second radius of curvature. The second curved segment 21 connects the first linear segment 20A with a second linear segment 22, and its curve is in a direction that is different than a direction of the curve of the first curved segment 20. More in particular, the second curved segment 21 comprises a concave portion that faces, or that is confronted with, an area/zone delimited by the first linear segment 20A and the second linear segment 22.The second linear segment 22 has a length which is larger than a length of the first linear segment 20A.

According to the proposed catheter 1, also, a third linear segment 23 is connected to an end of the second linear segment 22. Particularly, the connection is done by means of a curved portion 24 that purposely orients the third linear segment 23 in a direction that approaches towards the first curved segment 20. The radius of curvature of the curved portion 24 is particularly smaller than the first radius of curvature.

In some embodiments, the intersection of the third linear segment 23 and the second linear segment 22 covers an angle of at least 160º.

As shown in the illustrated embodiment, the second linear segment 22 and the first linear segment 20A are approximately parallel one to another. Likewise, the second radius of curvature is greater than the first radius of curvature.

In various embodiments, the overall length of the catheter 1 can be between 80 and 150 cm. The length of the second linear segment 22 can be between 3 and 10 cm. The length of the third linear segment 23 can be between 70 and 147 cm.

In some embodiments, the distal 10 or proximal 11 ends of the catheter 1, or other segments thereof, can be provided with a radiopaque element to enable visualization under fluoroscopy, or other imaging technique.

Unless otherwise indicated, all numbers expressing measurements, conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed patient matter.

As used herein, the term "about", when referring to a value or to an amount of a length, percentage, etc., is meant to encompass variations of in some embodiments ±10%, in some embodiments ±5%, in some embodiments ±1%, in some embodiments ±0.5%, and in some embodiments ±0.1% from the specified amount, as such variations are appropriate to perform the disclosed device.

Without further elaboration, it is believed that one skilled in the art can use the preceding description to utilize the invention to its fullest extent. It will be apparent to those having ordinary skill in the art, with the aid of the present disclosure, that changes may be made to the details of the above-described embodiments without departing from the underlying principles of the disclosure herein. In other words, various modifications and improvements of the embodiments specifically disclosed in the description above are within the scope of the appended claims. The scope of the invention is therefore defined by the following claims.

## Claims

1. A catheter for cerebral angiography and neurointerventions, comprising: a proximal end (11), a distal end (10) and a plurality of segments (20, 21, 22, 23), the plurality of segments including:
a first curved segment (20) having a curve with a first radius of curvature, the first curved segment (20) being extended with a first linear segment (20A);
a second curved segment (21) connecting the first linear segment (20A) with a second linear segment (22), the second curved segment (21) having a curve with a second radius of curvature,
the curve of the first curved segment (20) being in a different direction than the curve of the second curved segment (21 wherein
the plurality of segments (20, 21, 22, 23) further comprises a third linear segment (23) that is connected to an end of the second linear segment (22) through a curved portion (24) that orients the third linear segment (23) in a direction that approaches towards the first curved segment (20);
**characterised in that**
the second linear segment (22) having a length greater than a length of the first linear segment (22A).

2. The catheter of claim 1, wherein the curved portion (24) comprises a radius of curvature that is smaller than the first radius of curvature.

3. The catheter of claim 1 or 2, wherein the second linear segment (22) and the first linear segment (20A) are approximately parallel one to another.

4. The catheter of any one of the previous claims, wherein an intersection of the third linear segment (23) and the second linear segment (22) covers an angle of at least 160º.

5. The catheter of any one of the previous claims, wherein the first radius of curvature is smaller than the second radius of curvature.

6. The catheter of any one of the previous claims, wherein a total length of the catheter is between 80 cm and 150 cm.

7. The catheter of any one of the previous claims, wherein the second linear segment (22) has a length comprised in a range between 3 - 10 cm and the third linear segment (23) has a length comprised in a range between 70-147 cm.

## Patentansprüche

1. Katheter für zerebrale Angiografie und Neurointerventionen, umfassend: ein proximales Ende (11), ein distales Ende (10) und eine Vielzahl von Segmenten (20, 21, 22, 23), wobei die Vielzahl von Segmenten Folgendes umfasst:
ein erstes gekrümmtes Segment (20) mit einer Krümmung, die einen ersten Krümmungsradius aufweist, wobei das erste gekrümmte Segment (20) durch ein erstes lineares Segment (20A) verlängert wird;
ein zweites gekrümmtes Segment (21), das das erste lineare Segment (20A) mit einem zweiten linearen Segment (22) verbindet, wobei das zweite gekrümmte Segment (21) eine Krümmung mit einem zweiten Krümmungsradius aufweist,
wobei die Krümmung des ersten gekrümmten Segments (20) in eine andere Richtung verläuft als die Krümmung des zweiten gekrümmten Segments (21)
wobei
die Vielzahl von Segmenten (20, 21, 22, 23) ferner ein drittes lineares Segment (23) umfasst, das durch eine gekrümmte Stelle (24), die das dritte lineare Segment (23) zur Annäherung an das erste gekrümmte Segment (20) hin ausrichtet, mit einem Ende des zweiten linearen Segments (22) verbunden ist;
**dadurch gekennzeichnet, dass**
das zweite lineare Segment (22) eine Länge aufweist, die größer ist als die Länge des ersten linearen Segments (22A).

2. Katheter nach Anspruch 1, wobei die gekrümmte Stelle (24) einen Krümmungsradius aufweist, der kleiner ist als der erste Krümmungsradius.

3. Katheter nach Anspruch 1 oder 2, wobei das zweite lineare Segment (22) und das erste lineare Segment (20A) etwa parallel zueinander sind.

4. Katheter nach einem der vorstehenden Ansprüche, wobei ein Schnittpunkt des dritten linearen Segments (23) und des zweiten linearen Segments (22) einen Winkel von mindestens 160° bildet.

5. Katheter nach einem der vorstehenden Ansprüche, wobei der erste Krümmungsradius kleiner als der zweite Krümmungsradius ist.

6. Katheter nach einem der vorstehenden Ansprüche, wobei die Gesamtlänge des Katheters zwischen 80 cm und 150 cm liegt.

7. Katheter nach einem der vorstehenden Ansprüche, wobei das zweite lineare Segment (22) eine Länge im Bereich zwischen 3 und 10 cm und das dritte lineare Segment (23) eine Länge im Bereich zwischen 70 und 147 cm aufweist.

## Revendications

1. Cathéter pour angiographie cérébrale et neuro-interventions, comprenant : une extrémité proximale (11), une extrémité distale (10) et une pluralité de segments (20, 21, 22, 23), la pluralité de segments comportant :
un premier segment incurvé (20) ayant une courbe avec un premier rayon de courbure, le premier segment incurvé (20) étant étendu avec un premier segment linéaire (20A) ;
un deuxième segment incurvé (21) reliant le premier segment linéaire (20A) avec un deuxième segment linéaire (22), le deuxième segment incurvé (21) ayant une courbe avec un deuxième rayon de courbure,
la courbe du premier segment incurvé (20) étant dans une direction différente de la courbe du deuxième segment incurvé (21)
dans lequel
la pluralité de segments (20, 21, 22, 23) comprend en outre un troisième segment linéaire (23) qui est relié à une extrémité du deuxième segment linéaire (22) à travers une portion incurvée (24) qui oriente le troisième segment linéaire (23) dans une direction qui s'approche vers le premier segment incurvé (20) ;
**caractérisé en ce que**
le deuxième segment linéaire (22) présente une longueur supérieure à une longueur du premier segment linéaire (22A).

2. Cathéter selon revendication 1, dans lequel la portion incurvée (24) comprend un rayon de courbure qui est inférieur au premier rayon de courbure.

3. Cathéter selon revendication 1 ou 2, dans lequel le deuxième segment linéaire (22) et le premier segment linéaire (20A) sont approximativement parallèles l'un à l'autre.

4. Cathéter selon l'une quelconque des revendications précédentes, dans lequel une intersection du troisième segment linéaire (23) et du deuxième segment linéaire (22) couvre un angle d'au moins 160°.

5. Cathéter selon l'une quelconque des revendications précédentes, dans lequel le premier rayon de courbure est inférieur au deuxième rayon de courbure.

6. Cathéter selon l'une quelconque des revendications précédentes, dans lequel la longueur totale du cathéter est comprise entre 80 cm et 150 cm.

7. Cathéter selon l'une quelconque des revendications précédentes, dans lequel le deuxième segment linéaire (22) présente une longueur comprise dans une plage d'entre 3 - 10 cm et le troisième segment linéaire (23) présente une longueur comprise dans une plage d'entre 70-147 cm.
